# EUROPEAN PATENT SPECIFICATION

(11) **EP 1 820 446 B1**
(45) Date of publication and mention of the grant of the patent: **16.09.2009**
(21) Application number: 07102563.9
(22) Date of filing: 16.02.2007
(51) Int. Cl.: A61B 5/20

(54) **An apparatus for measuring body liquids**
Vorrichtung zur Messung von Körperflüssigkeiten
Appareil servant a mesurer des liquides corporels

(30) Priority: 16.02.2006 DK 200600222; 27.04.2006 DK 200600600
(43) Date of publication of application: 22.08.2007
(73) Proprietor: Unomedical A/S, 3460 Birkerød (DK)
(72) Inventor: James, Michael, SW17 0DF London (GB); Hansen, Trygve Kalf, 4040, Jyllinge (DK)
(74) Representative: Plougmann & Vingtoft A/S

(56) References cited:
- EP-A1- 0 471 413
- EP-A2- 0 983 746
- WO-A-89/05119
- WO-A-96/08219

## Description

The present invention relates to an apparatus for measuring and collecting volume of body liquids, which apparatus comprises a vessel with a liquid inlet at its top end and a liquid outlet at its lower end, and a measuring scale provided on the vessel for reading the volume of urine inside the vessel.

Apparatus of the kind mentioned above are used at hospitals for measuring and monitoring the urine discharge from bedridden patients, in particular from patients with catheters inserted into their urine bladder.

Various documents describe apparatuses for measuring urine, including EP 0 398 890, US 4,625,734, EP 0 008 450, US 6,482,190, EP 0 471 413, WO 96/08219 and US 4,178,934.

It is an object of the present invention to provide an apparatus for measuring and collecting urine, and which has an ergonomic rounded shape and a linear measuring scale.

It is a further object of the present invention to provide an apparatus for measuring and collecting urine, and which has a smooth outer shape being easier to keep clean.

### SUMMARY OF THE INVENTION

These objects are achieved with the apparatus according to the invention, the apparatus for measuring body liquids comprising a vessel with a liquid inlet at its top end and a liquid outlet at its lower end, the inside bottom surface of the vessel being curved, a measuring scale provided on the vessel for reading the volume of urine inside the vessel, and filling element(s) provided inside the vessel and taking up a predefined volume of the vessel to compensate for the inside curved bottom surface of the vessel in order to allow for the use of a linear measuring scale.

As the bottom surface is curved, the vessel obtains an ergonomic rounded shape which makes it easier to hold and handle for the nurses. However, as the bottom surface is curved, the measuring scale provided on the vessel can basically not be a linear scale, but in order to solve that, one or more filling elements are provided inside the vessel taking up a predefined volume of the vessel to compensate for the curved surface, which makes it possible to use a linear scale on the vessel.

The filling element(s) may be provided as projections or recesses in and/or a wall part of the vessel, such as for example on the back wall inside of the vessel, and it may define a straight horizontal bottom surface inside the vessel at a level substantially equal to the level at which the curved surface begins. The filling elements may be provided as projections moulded in a sidewall of the vessel during moulding of the vessel. "Wall part" means any bottom, top or side wall part of the vessel.

The filling element(s) may comprise independent fixed or movable elements arranged inside the vessel provided during or after manufacture of the vessel.

The curved bottom surface of the vessel allows for a better hygiene as there are no corners that can be difficult to access and clean from outside.

The curved bottom surface is preferably substantially circular formed, but it may have any curved form.

The apparatus may further comprise a valve provided with the outlet and a liquid collection bag connected to the liquid outlet and suspended from the measuring vessel.

A hollow valve body may be placed in and vertically displaceable with the measuring vessel, and the liquid outlet has the shape of a valve seat for the hollow valve body. The interior of the valve body is divided into a reception chamber and an overflow chamber, said reception chamber being connected to the liquid inlet and having at the lower end of the valve body at least one outlet opening which in the closed position of the valve body is closed by the valve seat and which at its upper part is connected to the measuring vessel through at least one hole in the chamber wall.

The overflow chamber may have at its lower end a duct which is directly connected to the measuring vessel via a hole in the chamber wall, the holes connecting the reception chamber to the measuring vessel and the measuring vessel to the overflow chamber being placed on the same side of the hollow valve body.

When the valve is in its closed position the outlet opening of the reception chamber is closed by the valve seat and consequently the liquid introduced into the reception chamber gradually fills said chamber which is preferably provided with the above mentioned linear measuring scale.

When the liquid levels with the hole in the chamber wall the introduction of additional liquid will cause overflow of liquid into the vessel, which vessel is thus gradually filled.

When the liquid surface in the vessel has reached the level of the hole in the overflow chamber the introduction of additional liquid will cause overflow into the overflow chamber and through the duct being in communication with the liquid outlet into the collection bag.

When emptying the reception chamber and vessel the valve body is lifted from the valve seat. This causes the liquid in the reception chamber to flow out through the outlet opening at the lower end of the chamber and allows the flow of liquid to pass from the vessel to the outlet and from here into the collection bag.

As the holes of the valve body are placed on the same side of the hollow valve body the apparatus may be positioned in an inclined position or horizontally provided, said holes face upwards without causing unintentional overflow of liquid from the reception chamber to the vessel and overflow chamber.

The valve body may have at its upper end a protrusion extending radially from the outside of the valve body which protrusion is placed in a helical groove on the inside of a surrounding part of the measuring vessel so as to cause a vertical displacement of the valve body by rotation of the valve body.

The valve body is preferably provided with a radially extending wing at its upper end to facilitate turning of the valve body.

The inside of the measuring vessel may comprise one or more protrusions which are in contact with the outside of the valve body and serve as a guide for same.

Two annular grooves for O-rings may be provided in that zone of the valve body which is in contact with the valve seat in its closed position in order to provide a liquid tight connection between the valve body and valve seat.

The valve body may be closed at the top by a cover which may comprise a liquid inlet in the form of a tubular member having an inclined end surface covered by a valve flap. The cover may comprise an opening which is covered by an air permeable and liquid tight filter which permits an outflow of air but not of liquid as the reception chamber and the vessel is filled with liquid and which permits an inflow of air when liquid is transferred to the collection bag.

### DESCRIPTION OF THE FIGURES

Embodiments of the Invention will now be described In details with reference to the accompanying figures, wherein
Figs. 1a-b show a vessel according to the invention,
Fig. 2 shows a vessel and a collection bag according to the invention,
Figs. 3a-b show the vessel of Figs. 1a-c more in detail, and
Figs. 4a-e show sectional views of the valve body of the vessel according to the invention.

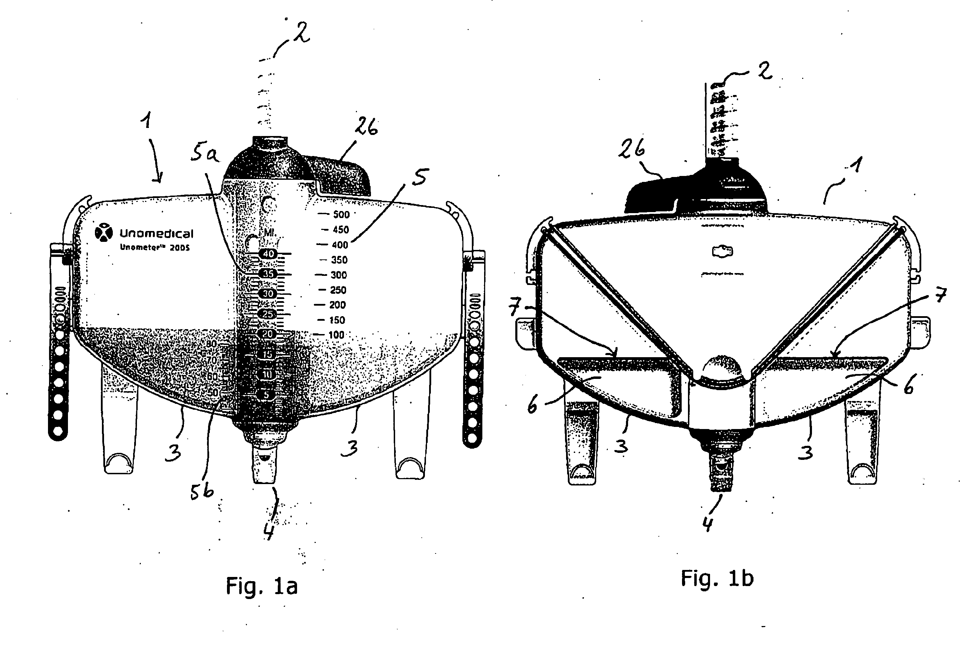

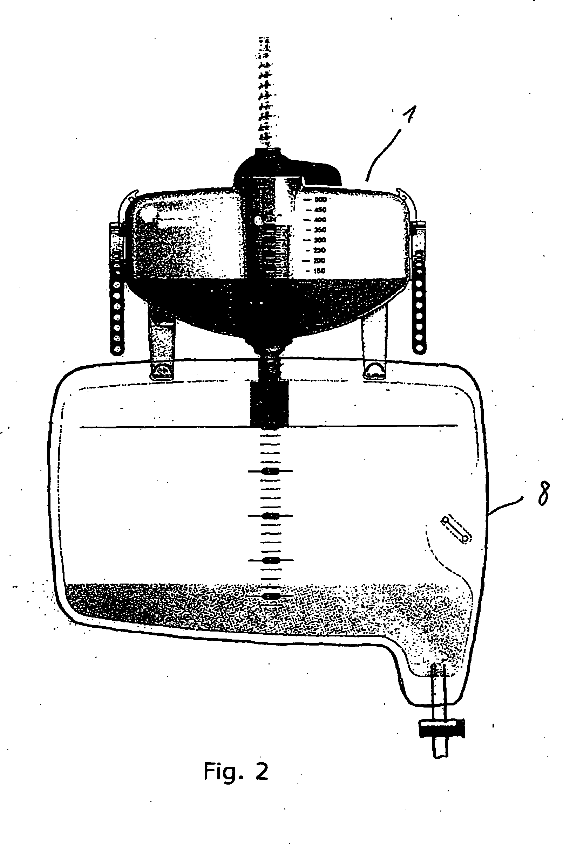

Figs. 1a-b show a front and back view of a vessel according to the invention for measuring volume of liquid, in particular urine from bedridden patients. The vessel 1 comprises a hollowed member with an inlet 2 for receiving and containing the liquid, and wherein the bottom surface 3 has a curved shape. An outlet 4 is provided in the bottom of the vessel 1, which can be coupled to a collection bag as shown in Fig. 2.

The vessel 1 is provided with a measuring scale 5 indicating the volume of liquid present therein. On the basis of a curved bottom surface 3, the measuring scale 5 can basically not be a linear scale, but in order to solve that problem, the vessel 1 is provided with filling elements 6 provided as recesses on its backside defining a straight horizontal bottom surface 7 at a level substantially equal to the level at which the curved surface begins. Due to the presence of the filling elements 6 taking up a predefined volume of the vessel and thereby compensating for the curved bottom surface 3, it Is possible to use a linear scale 5.

The curved surface 3 of the vessel 1 allows for a better hygiene as there are no corners that can be difficult to access and clean from outside. Furthermore, it provides a more ergonomic vessel being easier to hold.

Fig. 2 shows a vessel 1 and a collection bag 8 connected to each other via the outlet 4 of the vessel and an inlet of the bag, which may be formed by a tubular member being adhered to the bag 8.

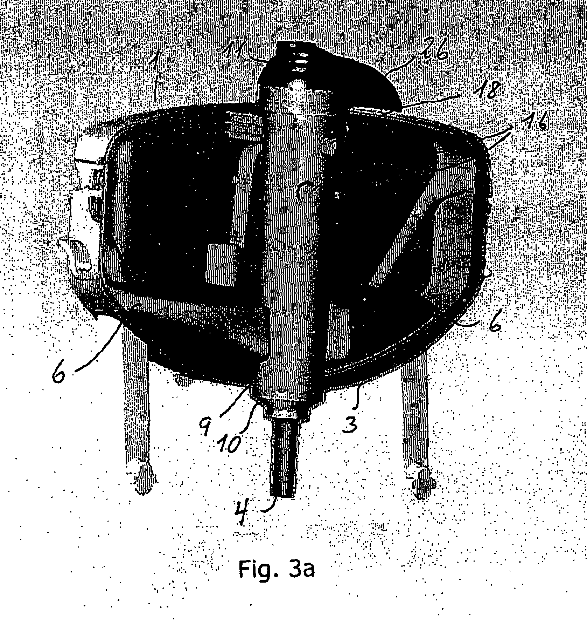

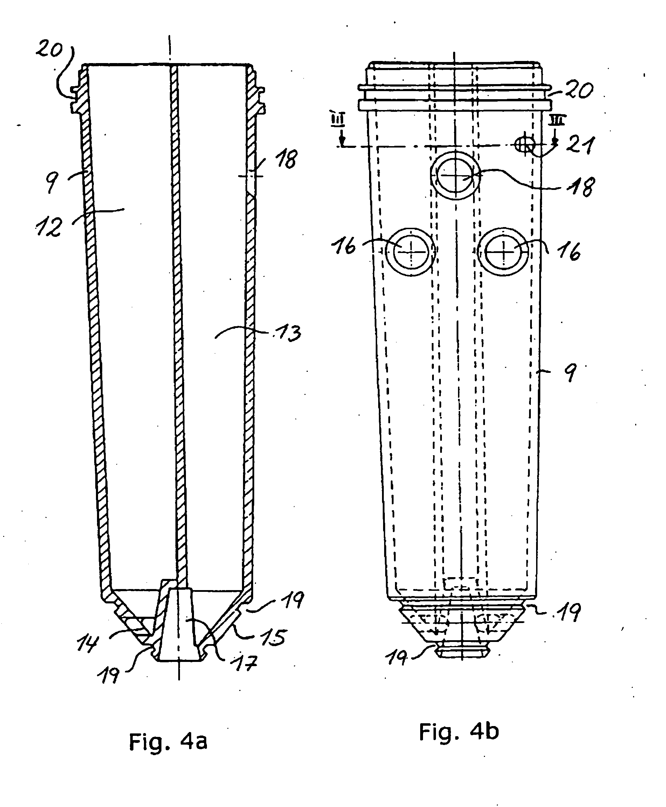

Figs. 3a-b show transparent views of the vessel of Figs. 1a-b and 2 In order to see the inside of the vessel more in detail. The vessel 1 comprises the hollowed member for receiving and containing the liquid, and the bottom surface 3 has a curved shape provided on each side of a central column terminating in the outlet 4 of the vessel 4. The central column defines a valve body 9 for the vessel, the valve body 9 being axially displaceable by rotation and which at its lower end is in contact with a valve seat 10 and is closed at the top with a cover 11. The valve body 9 is described more in detail with reference to Figs. 4a-e.

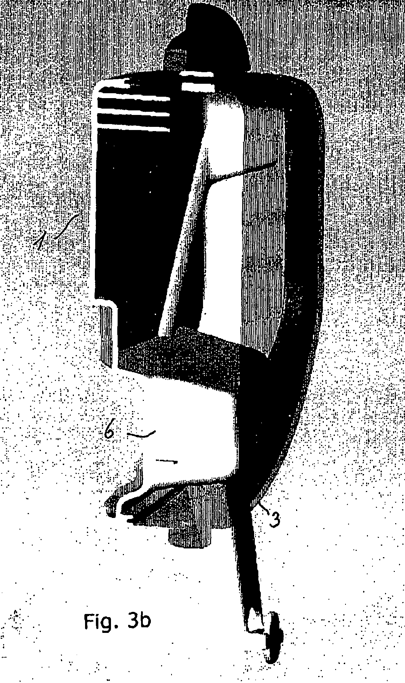

Fig. 3b is a cross-sectional view of the vessel 1. As seen in fig. 3b, the filling element 6 is provided as a projection moulded in the back wall of the vessel.

Figs. 4a-e show sectional views of a valve body 9 of the vessel 1 as shown in Figs. 1a-b, Fig. 2 and Figs. 3a-b.

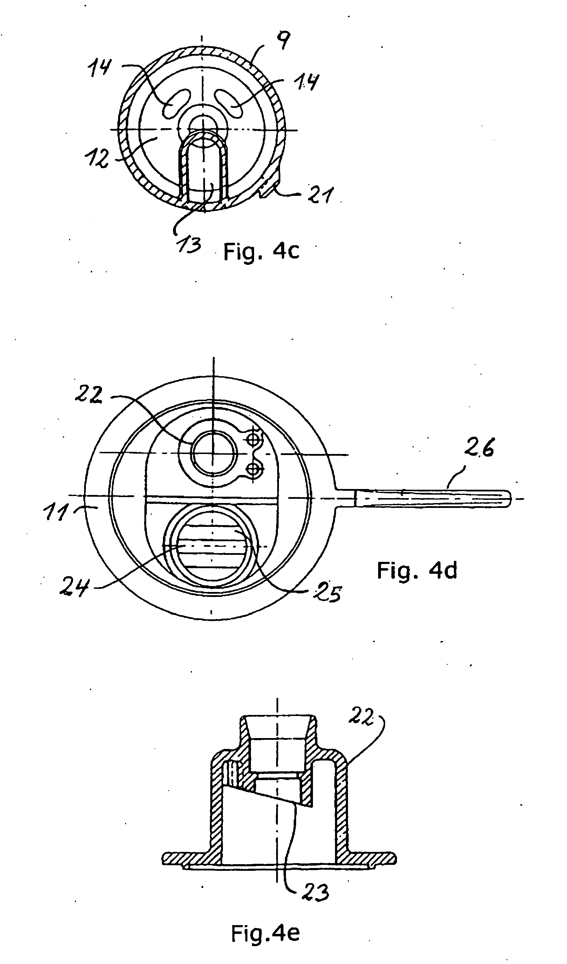

Fig. 4c is a sectional view along the line III-III of the valve body of Fig. 4a, Fig. 4d shows the upper part of the valve body seen from the bottom, and Fig. 4e shows the upper part of the valve body of Fig. 4d in sectional view.

The central column defines the valve body 9, which is constructed with two chambers; a reception chamber 12 and an overflow chamber 13. At its lower end the reception chamber 12 is provided with two holes 14 ending in a valve surface 15 which contacts the valve seat 10 in its closed position thus preventing liquid outflow. At the upper end of the reception chamber 12 two holes 16 are provided in the wall separating the chamber 12 from the surrounding vessel 1.

At its lower end the overflow chamber 13 is provided with a duct 17 through which said overflow chamber 13 is in direct contact with the liquid outlet of the vessel. The liquid outlet is defined by a tubular member so that the vessel can be connected to a collection bag 8.

At the top end of the overflow chamber 13 a hole 18 is provided in the valve wall.

Two annular-shaped grooves 19 each comprising an O-ring (not shown) is provided on the outside of the valve body at the lower end thereof. An annular groove 20 comprising an O-ring (not shown) is provided.

The outside of the valve body 9 is also provided with a projection 21 being inserted in a helical groove formed on the outside of the back wall of the measuring vessel 1.

The cover 11 of the valve body 9 is provided with two openings one being a tubular member 22 ending in an inclined surface 23 covered by a rubber flap (not shown) permitting the introduction of liquid and preventing back flow of the same. A ventilation opening 24 is provided which is partially covered by support ribs 25 supporting an air filter (not shown).

The liquid passes down into the reception chamber 12 inside the valve body 9 via the tubular member 22. In its initial (closed) position the valve surface 15 of the valve body 9 is in contact with the valve seat 10 and consequently no liquid is allowed to pass through the holes 14. Therefore, the liquid level in the reception chamber 12 will rise and using the measuring scale 5a on the valve body 9, the volume of the collected liquid may be read.

When the liquid surface has reached the level of the holes 16, the introduction of additional liquid will start filling of the vessel 1.

The vessel is provided with an additional non-linear measuring scale 5b in the lower end of the vessel with the curved bottom surface to measure a first amount of liquid entering the vessel from the reception chamber. Due to the presence of the filling elements 6 compensating for the curved bottom surface, the measuring scale goes from the non-linear scale 5b to the linear scale 5.

If additional amounts of liquid are introduced the vessel 1 is also filled and through the hole 18 an overflow of liquid from the vessel 1 in the overflow chamber 13 may occur. From here the liquid passes via a duct 17 down through the liquid outlet 6 and into a collection bag 8.

At this time or at any earlier desired time the reception chamber 12 as well as the vessel 1 may be emptied by clockwise rotation of the wing 26. Such rotation causes the valve body 9 to be rotated and because the projection 21 is located in an upwards slanting groove (not shown) the valve body 9 is lifted upwards in connection with the rotation thus removing the valve surface 15 from the valve seat 10. Hence it is possible to empty the reception chamber quickly through the holes 14 and the vessel 1 through the space between the valve surface 15 and the valve seat 10 as liquid flows down into the collection bag 8 being connected to the outlet.

## Claims

1. An apparatus for measuring body liquids comprising;
- a measuring vessel (1) with a liquid inlet (2) at its top end and a liquid outlet (4) at its lower end, the inside bottom surface (3) of the vessel (1) being curved,
- a measuring scale (5) provided on the vessel (1) for reading the volume of urine inside the vessel (1), **characterized in that** it further comprises
- filling elements (6) provided inside the vessel (1) and taking up a predefined volume of the vessel (1) to compensate for the inside curved bottom surface (3) of the vessel (1) in order to allow for the use of a linear measuring scale (5).

2. An apparatus according to claim 1, wherein the filling elements (6) comprise projections or recesses (6) provided in and/or on a wall part inside of the vessel (1).

3. An apparatus according to claim 1 or 2, wherein the filling elements comprise independent fixed or movable elements arranged inside the vessel after manufacture of the vessel.

4. An apparatus according to any of claims 1-3, wherein the bottom surface (3) is formed as a circular arc.

5. An apparatus according to any of claims 1-4, further comprising a valve (9, 10) provided with the outlet (4) and a liquid collection bag (8) connected to the liquid (4) outlet and suspended from the measuring vessel (1).

6. An apparatus according to claim 5 and comprising a hollow valve body (9) placed in and vertically displaceable with the measuring vessel (1), the liquid outlet (4) having the shape of a valve seat (10) for the hollow valve body (9), the interior of the valve body (9) being divided into a reception chamber (12) and an overflow chamber (13), said reception chamber (12) being connected to the liquid inlet (2) and having at the lower end of the valve body (9) at least one outlet opening (14) which in the closed position of the valve body (9) is closed by the valve seat (10) and which at its upper part is connected to the measuring vessel (1) through at least one hole (16) in the chamber wall, the overflow chamber (13) having at its lower end a duct (17) which is directly connected to the measuring vessel (1) via a hole (18) in the chamber wall, the holes connecting the reception chamber (12) to the measuring vessel (1) and the measuring vessel (1) to the overflow chamber (13) being placed on the same side of the hollow valve body (9).

7. An apparatus according to claim 6, wherein the valve body (9) has at its upper end a protrusion (21) extending radially from the outside of the valve body (9) which protrusion (21) is placed in a helical groove on the inside of a surrounding part of the measuring vessel (1) so as to cause a vertical displacement of the valve body (9) by rotation of the valve body (9).

8. An apparatus according to claim 7, wherein the valve body (9) is provided with a radially extending wing (26) at its upper end.

9. An apparatus according to any of claims 6-8, wherein the inside of the measuring vessel (1) comprises one or more protrusions which are in contact with the outside of the valve body (9) and serve as a guide for same.

10. An apparatus according to any of claims 6-9, wherein two annular grooves (19) for O-rings are provided in that zone of the valve body (9) which is in contact with the valve seat (10) in its closed position.

11. An apparatus according to any of claims 6-10, wherein the valve body (9) is closed at the top by a cover (11).

12. An apparatus according to claim 11, wherein the cover (11) comprises a liquid inlet (22) in the form of a tubular member having an inclined end surface covered by a valve flap.

13. An apparatus according to claim 12, wherein the cover (11) comprises an opening (24) which is covered by an air permeable and liquid tight filter.

14. An apparatus according to any of the preceding claims for measuring and collecting urine.

## Patentansprüche

1. Gerät zum Messen von Körperflüssigkeiten, umfassend;
- einen Messbehälter (1) mit einem Flüssigkeitseinlass (2) im oberen Ende und einem Flüssigkeitsauslass (4) im unteren Ende, wobei die innere Bodenfläche (3) des Behälters (1) gekrümmt ist,
- eine Messskala (5) auf dem Behälter (1) zum Ablesen des Urinvolumens innerhalb des Behälters (1), **dadurch gekennzeichnet, dass** es weiterhin
- Füllelemente (6) innerhalb des Behälters (1) umfasst, die ein vorbestimmtes Volumen des Behälters (1) aufnehmen, um für die innere gekrümmte Bodenfläche (3) des Behälters (1) zu kompensieren, um die Anwendung einer linearen Messskala (5) zu ermöglichen.

2. Gerät nach Anspruch 1, wo die Füllelemente (6) Vorsprünge oder Aussparungen (6), die in und/oder auf einem Teil der Wand im Behälter (1) vorgesehen sind, umfassen.

3. Gerät nach Anspruch 1 oder 2, wo die Füllelemente unabhängige befestigte oder bewegliche Elemente, die nach der Herstellung des Behälters im Behälter angebracht sind, umfassen.

4. Gerät nach irgendeinem der Ansprüche 1-3, wo die Bodenfläche (3) als ein kreisförmiger Bogen ausgeformt ist.

5. Gerät nach irgendeinem der Ansprüche 1-4, weiterhin umfassend ein Ventil (9, 10), das mit dem Auslass (4) und einem Flüssigkeitssammelbeutel (8), der mit dem Flüssigkeitsauslass (4) verbunden ist und am Messbehälter (1) aufgehängt ist, versehen ist.

6. Gerät nach Anspruch 5, umfassend einen hohlen Ventilkörper (9), der im Messbehälter (1) angebracht und damit vertikal verlagert ist, wobei der Flüssigkeitsauslass (4) die Form eines Ventilsitzes (10) für den hohlen Ventilkörper hat, wobei das Innere des Ventilkörpers (9) in eine Aufnahmekammer (12) und eine Überlaufkammer (13) aufgeteilt ist, wo die Aufnahmekammer (12) mit dem Flüssigkeitseinlass (2) verbunden ist und im unteren Ende des Ventilkörpers (9) mindestens eine Auslassöffnung (14) hat, die in der geschlossenen Position des Ventilkörpers (9) durch den Ventilsitz (10) geschlossen wird, und die bei ihrem oberen Teil mit dem Messbehälter (1) durch mindestens eine Bohrung (16) in der Kammerwand verbunden ist, wobei die Überlaufkammer (13) in ihrem unteren Ende einen Durchgang (17) hat, der mit dem Messbehälter (1) durch eine Bohrung (18) in der Kammerwand direkt verbunden ist, wobei die Bohrungen die die Aufnahmekammer (12) mit dem Messbehälter (1) und den Messbehälter (1) mit der Überlaufkammer (13) verbinden, auf der selben Seite des hohlen Ventilkörpers (9) angebracht sind.

7. Gerät nach Anspruch 6, wo der Ventilkörper (9) in seinem oberen Ende einen Überstand (21) hat, der sich radial von der Außenseite des Ventilkörpers (9) erstreckt, welcher Überstand (21) in einer spiralförmigen Nut auf der Innenseite eines umgebenden Teils des Messbehälters (1) angebracht ist, um eine vertikale Verlagerung des Ventilkörpers (9) durch Rotation des Ventilkörpers (9) zu verursachen.

8. Gerät nach Anspruch 7, wo der Ventilkörper (9) mit einem sich radial erstreckenden Flügel (26) in seinem oberen Ende versehen ist.

9. Gerät nach irgendeinem der Ansprüche 6-8, wo die Innenseite des Messbehälters (1) einen oder mehrere Überstände umfasst, die mit der Außenseite des Ventilkörpers (9) in Kontakt stehen und als eine Führung dafür dienen.

10. Gerät nach irgendeinem der Ansprüche 6-9, wo zwei ringförmige Nuten (19) für O-Ringe in der Zone des Ventilkörpers (9), der mit dem Ventilsitz (10) in seiner geschlossenen Position in Kontakt steht, vorgesehen sind.

11. Gerät nach irgendeinem der Ansprüche 6-10, wo der Ventilkörper (9) oben durch einen Deckel (11) geschlossen ist.

12. Gerät nach Anspruch 11, wo der Deckel (11) einen Flüssigkeitseinlass (22) in Form eines rohrförmigen Elements mit einer schrägen durch eine Ventilklappe zugedeckte Endfläche umfasst.

13. Gerät nach Anspruch 12, wo der Deckel (11) eine Öffnung (24), die durch ein luftdurchlässiges und flüssigkeitsdichtes Filter zugedeckt ist, umfasst.

14. Gerät nach irgendeinem der vorhergehenden Ansprüche zum Messen und Aufsammeln von Urin.

## Revendications

1. Un appareil de mesure des liquides corporels comprenant:
- un récipient de mesure (1) ayant une entrée de liquide (2) à son extrémité supérieure et une sortie de liquide (4) à son extrémité inférieure, la partie intérieure de la surface inférieure (3) du récipient (1) étant courbée,
- une échelle de mesure (5) fournie sur le récipient (1) pour lire le volume d'urine à l'intérieur du récipient (1), **caractérisé en ce qu'**il comprend en outre
- des éléments de remplissage (6) fournis à l'intérieur du récipient (1) qui occupent un volume prédéfini du récipient (1) pour compenser la partie intérieure courbée de la surface inférieure (3) du récipient (1) afin de permettre l'utilisation d'une échelle de mesure linéaire (5).

2. Un appareil selon la revendication 1, **caractérisé en ce que** les éléments de remplissage (6) comprennent des saillies ou des cavités (6) dans et/ou sur une partie de la paroi intérieure du récipient (1).

3. Un appareil selon les revendications 1 ou 2, **caractérisé en ce que** les éléments de remplissage comprennent des éléments indépendants fixes ou amovibles agencés à l'intérieur du récipient après fabrication du récipient.

4. Un appareil selon l'une quelconque des revendications 1 à 3, **caractérisé en ce que** la surface inférieure (3) a la forme d'un arc circulaire.

5. Un appareil selon l'une quelconque des revendications 1 à 4, comprenant en outre une vanne (9, 10) munie d'une sortie (4) et un réservoir de récupération de liquide (8) connecté à la sortie du liquide (4) et suspendu au récipient de mesure (1).

6. Un appareil selon la revendication 5 et comprenant un corps de vanne creux (9) placé dans et verticalement déplaçable avec le récipient de mesure (1), la sortie de liquide (4) ayant la forme d'une portée de vanne (10) pour le corps de vanne creux (9), l'intérieur du corps de vanne (9) étant divisé en une chambre de réception (12) et une chambre de débordement (13), ladite chambre de réception (12) étant connectée à l'entrée de liquide (2) et ayant au niveau de la partie inférieure du corps de vanne (9) au moins un orifice de sortie (14) qui en position fermée du corps de la vanne (9) est fermé par la portée de vanne (10), et qui au niveau de sa partie supérieure est connectée au récipient de mesure (1) par au moins un trou (16) sur la paroi de la chambre, la chambre de débordement (13) ayant au niveau de sa partie inférieure une conduite (17) qui est connectée directement au récipient de mesure (1) par un trou (18) sur la paroi de la chambre, les trous reliant la chambre de réception (12) au récipient de mesure (1) et le récipient de mesure (1) à la chambre de débordement (13) étant placés du même côté du corps de vanne creux (9).

7. Un appareil selon la revendication 6, **caractérisé en ce que** le corps de vanne (9) a au niveau de son extrémité supérieure une saillie (21) qui s'étend radialement de l'extérieur du corps de vanne (9), laquelle saillie (21) est placée dans un sillon hélicoïdal à l'intérieur d'une partie entourant le récipient de mesure (1) afin de créer un déplacement vertical du corps de vanne (9) par rotation du corps de vanne (9).

8. Un appareil selon la revendication 7, **caractérisé en ce que** le corps de vanne (9) est muni d'une aile d'extension radiale (26) au niveau de son extrémité supérieure.

9. Un appareil selon l'une quelconque des revendications 6 à 8, **caractérisé en ce que** l'intérieur du récipient de mesure (1) comprend une ou plusieurs saillies qui sont en contact avec l'extérieur du corps de vanne (9) et servent de guide pour le récipient.

10. Un appareil selon l'une quelconque des revendications 6 à 9, **caractérisé en ce que** deux sillons annulaires (19) pour des joints toriques sont fournis dans la zone du corps de vanne (9) qui est en contact avec la portée de vanne (10) en position fermée.

11. Un appareil selon l'une quelconque des revendications 6 à 10, **caractérisé en ce que** le corps de vanne (9) est fermé en haut par un couvercle (11).

12. Un appareil selon la revendication 11, **caractérisé en ce que** le couvercle (11) comprend un point d'entrée (22) de la forme d'un membre tubulaire ayant une extrémité de surface inclinée recouverte par un clapet battant.

13. Un appareil selon la revendication 12, **caractérisé en ce que** le couvercle (11) comprend une ouverture (24) qui est recouverte par un filtre perméable à l'air et étanche aux liquides.

14. Un appareil selon l'une quelconque des revendications précédentes pour mesurer et récupérer l'urine.
